# EUROPEAN PATENT APPLICATION

(11) **EP 1 281 756 A1**
(43) Date of publication of application: **05.02.2003**
(21) Application number: 01118401.7
(22) Date of filing: 31.07.2001
(51) Int. Cl.: C12N 9/16, A61K 38/00, G01N 33/573, C12Q 1/68

(54) **Regulator of calcineurin**

(71) Applicant: GENOPIA Biomedical GmbH, 66123 Saarbrücken (DE)
(72) Inventor: Völkel, Helge, Dr., 80981 Ulm (DE)
(74) Representative: Patentanwälte Ruff, Wilhelm, Beier, Dauster & Partner

(57) **Abstract**

A regulator of Calcineurin is provided, which is based on a splice variant of the catalytic subunit of Calcineurin. Preferably the catalytic subunit is the β-form, γ-form and/or α-form of this subunit. The invention comprises the regulator and a substance, which influences the stimulating activity of the regulator, as active agents for the treatment of diseases, which are preferably connected with a disturbance of Calcineurin activity.

SEQUENCE LISTING

<110> GENOPIA Biomedical GmbH

<120> Regulator of Calcineurin

<130> P 41 239 EP

<160> 16

<170> PatentIn version 3.1

<210> 1
<211> 474
<212> DNA
<213> Homo sapiens

<220>
<221> misc_feature
<223> CNA beta splice variant (a)

<400> 1
atggccgccc cggagccggc ccgggctgca ccgcccccac ccccgccccc gccgccccct 60
cccggggctg accgcgtcgt caaaggttca gctgcagccc ggaaagaaat cataagaaac 120
aaaattcgag caattggcaa gatggcaaga gtcttctctg ttctcaggga ggagagtgaa 180
agtgtgctga cactcaaggg cctgactccc acagggatgt tgcctagtgg agtgttagct 240
ggaggacggc agaccctgca aagtgccaca gttgaggcta ttgaggctga aaaagcaata 300
cgaggattct ctccaccaca tagaatctgc agttttgaag aggcaaaggg tttggatagg 360
atcaatgaga gaatgccacc tcggaaagat gctgtacagc aagatggttt caattctctg 420
aacaccgcac atgccactga gaaccacggg acgggcaacc atactgccca gtga 474

<210> 2

<211>  444

<212>  DNA

<213>  Homo sapiens

<220>

<221>  misc_feature

<223>  CNA beta splice variant (b)

<400>  2
atggccgccc cggagccggc ccgggctgca ccgcccccac ccccgccccc gccgccccct      60

cccggggctg accgcgtcgt caaaggttca gctgcagccc ggaaagaaat cataagaaac     120

aaaattcgag caattggcaa gatggcaaga gtcttctctg ttctcaggga ggagagtgaa     180

agtgtgctga cactcaaggg cctgactccc acagggatgt tgcctagtgg agtgttagct     240

ggaggacggc agaccctgca aagtgccata cgaggattct ctccaccaca tagaatctgc     300

agttttgaag aggcaaaggg tttggatagg atcaatgaga gaatgccacc tcggaaagat     360

gctgtacagc aagatggttt caattctctg aacaccgcac atgccactga gaaccacggg     420

acgggcaacc atactgccca gtga                                            444

<210>  3

<211>  447

<212>  DNA

<213>  Homo sapiens

<220>

<221>  misc_feature

<223>  CNA gamma splice variant (a)

<400>  3
atgtccggga ggcgcttcca cctctccacc accgaccgcg tcatcaaagg aagcactaca      60

gttcgtaagg agatcatcag gaataagatc agagccattg ggaagatggc acgggtcttt     120

tcaattcttc ggcaagaaag tgagagtgtg ctgactctca agggcctgac tcccacaggc     180

acactccctc tgggcgtcct ctcaggaggc aagcagacta tcgagacagc cacagtagaa     240

gcggtagagg cccgggaagc catcagaggg ttctcgcttc agcacaagat ccggagtttt     300

gaagaagcgc gaggtctgga ccgaattaat gagcgaatgc caccccgaaa ggatagcata 360

taccctggtg ggccaatgaa atctgtaacc tcagcacact cacatgctgc gcacaggagc 420

gaccaaggga agaaagccca ttcatga 447

<210> 4

<211> 417

<212> DNA

<213> Homo sapiens

<220>

<221> misc_feature

<223> CNA gamma splice variant (b)

<400> 4
atgtccggga ggcgcttcca cctctccacc accgaccgcg tcatcaaagg aagcactaca 60

gttcgtaagg agatcatcag gaataagatc agagccattg ggaagatggc acgggtcttt 120

tcaattcttc ggcaagaaag tgagagtgtg ctgactctca agggcctgac tcccacaggc 180

acactccctc tgggcgtcct ctcaggaggc aagcagacta tcgagacagc catcagaggg 240

ttctcgcttc agcacaagat ccggagtttt gaagaagcgc gaggtctgga ccgaattaat 300

gagcgaatgc caccccgaaa ggatagcata taccctggtg ggccaatgaa atctgtaacc 360

tcagcacact cacatgctgc gcacaggagc gaccaaggga agaaagccca ttcatga 417

<210> 5

<211> 468

<212> DNA

<213> Homo sapiens

<220>

<221> misc_feature

<223> CNA alpha splice variant (a)

<400> 5
atgtccgagc ccaaggcaat tgatcccaag ttgtcgacga ccgacagggt ggtgaaaggt 60

gcaacagctg cagcccggaa agaggcgata aggaacaaga tccgagcaat aggcaaaatg 120

gccagagtgt tctcagtgct cagagaagag agtgagagtg tgctgacgct gaaaggcttg 180

accccaactg gcatgctccc cagcggagta ctctctggag ggaagcaaac cctgcaaagc 240

gctactgttg aggctattga ggctgatgaa gctatcaaag gattttcacc acaacataag 300

atcactagct tcgaggaagc caagggctta gaccgaatta atgagaggat gccgcctcgc 360

agagatgcca tgccctctga cgccaacctt aactccatca acaaggctct cacctcagag 420

actaacggca cggacagcaa tggcagtaat agcagcaata ttcagtga 468

<210> 6

<211> 438

<212> DNA

<213> Homo sapiens

<220>

<221> misc_feature

<223> CNA alpha splice variant (b)

<400> 6
atgtccgagc ccaaggcaat tgatcccaag ttgtcgacga ccgacagggt ggtgaaaggt 60

gcaacagctg cagcccggaa agaggcgata aggaacaaga tccgagcaat aggcaaaatg 120

gccagagtgt tctcagtgct cagagaagag agtgagagtg tgctgacgct gaaaggcttg 180

accccaactg gcatgctccc cagcggagta ctctctggag ggaagcaaac cctgcaaagc 240

gctatcaaag gattttcacc acaacataag atcactagct tcgaggaagc caagggctta 300

gaccgaatta atgagaggat gccgcctcgc agagatgcca tgccctctga cgccaacctt 360

aactccatca acaaggctct cacctcagag actaacggca cggacagcaa tggcagtaat 420

agcagcaata ttcagtga 438

<210> 7

<211> 157

<212> PRT

<213> Homo sapiens

<220>

<221>  MISC_FEATURE

<223>  CNA beta splice variant (a)

<400>  7

Met Ala Ala Pro Glu Pro Ala Arg Ala Ala Pro Pro Pro Pro Pro Pro
1               5                   10                  15

Pro Pro Pro Pro Pro Gly Ala Asp Arg Val Val Lys Gly Ser Ala Ala
            20                  25                  30

Ala Arg Lys Glu Ile Ile Arg Asn Lys Ile Arg Ala Ile Gly Lys Met
        35                  40                  45

Ala Arg Val Phe Ser Val Leu Arg Glu Glu Ser Glu Ser Val Leu Thr
    50                  55                  60

Leu Lys Gly Leu Thr Pro Thr Gly Met Leu Pro Ser Gly Val Leu Ala
65                  70                  75                  80

Gly Gly Arg Gln Thr Leu Gln Ser Ala Thr Val Glu Ala Ile Glu Ala
                85                  90                  95

Glu Lys Ala Ile Arg Gly Phe Ser Pro Pro His Arg Ile Cys Ser Phe
            100                 105                 110

Glu Glu Ala Lys Gly Leu Asp Arg Ile Asn Glu Arg Met Pro Pro Arg
        115                 120                 125

Lys Asp Ala Val Gln Gln Asp Gly Phe Asn Ser Leu Asn Thr Ala His
    130                 135                 140

Ala Thr Glu Asn His Gly Thr Gly Asn His Thr Ala Gln
145                 150                 155

<210>  8

<211>  147

<212>  PRT

<213>  Homo sapiens

<220>

<221>  MISC_FEATURE

<223>  CNA beta splice variant (b)

<400>  8

Met Ala Ala Pro Glu Pro Ala Arg Ala Ala Pro Pro Pro Pro Pro Pro
1               5                   10                  15

Pro Pro Pro Pro Pro Gly Ala Asp Arg Val Val Lys Gly Ser Ala Ala
            20                  25                  30

Ala Arg Lys Glu Ile Ile Arg Asn Lys Ile Arg Ala Ile Gly Lys Met
        35                  40                  45

Ala Arg Val Phe Ser Val Leu Arg Glu Glu Ser Glu Ser Val Leu Thr
    50                  55                  60

Leu Lys Gly Leu Thr Pro Thr Gly Met Leu Pro Ser Gly Val Leu Ala
65                  70                  75                  80

Gly Gly Arg Gln Thr Leu Gln Ser Ala Ile Arg Gly Phe Ser Pro Pro
                85                  90                  95

His Arg Ile Cys Ser Phe Glu Glu Ala Lys Gly Leu Asp Arg Ile Asn
            100                 105                 110

Glu Arg Met Pro Pro Arg Lys Asp Ala Val Gln Gln Asp Gly Phe Asn
        115                 120                 125

Ser Leu Asn Thr Ala His Ala Thr Glu Asn His Gly Thr Gly Asn His
    130                 135                 140

Thr Ala Gln
145

<210>  9

<211>  148

<212>  PRT

<213>  Homo sapiens

<220>

<221>  MISC_FEATURE

<223>  CNA gamma splice variant (a)

<400>  9

Met Ser Gly Arg Arg Phe His Leu Ser Thr Thr Asp Arg Val Ile Lys
1               5                   10                  15

Gly Ser Thr Thr Val Arg Lys Glu Ile Ile Arg Asn Lys Ile Arg Ala
            20                  25                  30

Ile Gly Lys Met Ala Arg Val Phe Ser Ile Leu Arg Gln Glu Ser Glu
        35                  40                  45

Ser Val Leu Thr Leu Lys Gly Leu Thr Pro Thr Gly Thr Leu Pro Leu
    50                  55                  60

Gly Val Leu Ser Gly Gly Lys Gln Thr Ile Glu Thr Ala Thr Val Glu
65                  70                  75                  80

Ala Val Glu Ala Arg Glu Ala Ile Arg Gly Phe Ser Leu Gln His Lys
                85                  90                  95

Ile Arg Ser Phe Glu Glu Ala Arg Gly Leu Asp Arg Ile Asn Glu Arg
            100                 105                 110

Met Pro Pro Arg Lys Asp Ser Ile Tyr Pro Gly Gly Pro Met Lys Ser
        115                 120                 125

Val Thr Ser Ala His Ser His Ala Ala His Arg Ser Asp Gln Gly Lys
    130                 135                 140

Lys Ala His Ser
145

<210>  10

<211>  138

<212>  PRT

<213>  Homo sapiens

<220>

<221>  MISC_FEATURE

<223>  CNA gamma splice variant (b)

<400>  10

Met Ser Gly Arg Arg Phe His Leu Ser Thr Thr Asp Arg Val Ile Lys

1               5                   10                  15

Gly Ser Thr Thr Val Arg Lys Glu Ile Ile Arg Asn Lys Ile Arg Ala
            20                  25                  30

Ile Gly Lys Met Ala Arg Val Phe Ser Ile Leu Arg Gln Glu Ser Glu
        35                  40                  45

Ser Val Leu Thr Leu Lys Gly Leu Thr Pro Thr Gly Thr Leu Pro Leu
    50                  55                  60

Gly Val Leu Ser Gly Gly Lys Gln Thr Ile Glu Thr Ala Ile Arg Gly
65                  70                  75                  80

Phe Ser Leu Gln His Lys Ile Arg Ser Phe Glu Glu Ala Arg Gly Leu
                85                  90                  95

Asp Arg Ile Asn Glu Arg Met Pro Pro Arg Lys Asp Ser Ile Tyr Pro
            100                 105                 110

Gly Gly Pro Met Lys Ser Val Thr Ser Ala His Ser His Ala Ala His
        115                 120                 125

Arg Ser Asp Gln Gly Lys Lys Ala His Ser
    130                 135

<210>  11

<211>  155

<212>  PRT

<213>  Homo sapiens

<220>

<221>  MISC_FEATURE

<223>  CNA alpha splice variant (a)

<400>  11

Met Ser Glu Pro Lys Ala Ile Asp Pro Lys Leu Ser Thr Thr Asp Arg
1               5                   10                  15

Val Val Lys Gly Ala Thr Ala Ala Ala Arg Lys Glu Ala Ile Arg Asn
            20                  25                  30

Lys Ile Arg Ala Ile Gly Lys Met Ala Arg Val Phe Ser Val Leu Arg
            35                  40                  45

Glu Glu Ser Glu Ser Val Leu Thr Leu Lys Gly Leu Thr Pro Thr Gly
    50                  55                  60

Met Leu Pro Ser Gly Val Leu Ser Gly Gly Lys Gln Thr Leu Gln Ser
65                  70                  75                  80

Ala Thr Val Glu Ala Ile Glu Ala Asp Glu Ala Ile Lys Gly Phe Ser
                85                  90                  95

Pro Gln His Lys Ile Thr Ser Phe Glu Glu Ala Lys Gly Leu Asp Arg
            100                 105                 110

Ile Asn Glu Arg Met Pro Pro Arg Arg Asp Ala Met Pro Ser Asp Ala
        115                 120                 125

Asn Leu Asn Ser Ile Asn Lys Ala Leu Thr Ser Glu Thr Asn Gly Thr
    130                 135                 140

Asp Ser Asn Gly Ser Asn Ser Ser Asn Ile Gln
145                 150                 155

<210>  12

<211>  145

<212>  PRT

<213>  Homo sapiens

<220>

<221>  MISC_FEATURE

<223>  CNA alpha splice variant (b)

<400>  12

Met Ser Glu Pro Lys Ala Ile Asp Pro Lys Leu Ser Thr Thr Asp Arg
1               5                   10                  15

Val Val Lys Gly Ala Thr Ala Ala Ala Arg Lys Glu Ala Ile Arg Asn
            20                  25                  30

Lys Ile Arg Ala Ile Gly Lys Met Ala Arg Val Phe Ser Val Leu Arg
        35                  40                  45

Glu Glu Ser Glu Ser Val Leu Thr Leu Lys Gly Leu Thr Pro Thr Gly
    50                  55                  60

Met Leu Pro Ser Gly Val Leu Ser Gly Gly Lys Gln Thr Leu Gln Ser
65                  70                  75                  80

Ala Ile Lys Gly Phe Ser Pro Gln His Lys Ile Thr Ser Phe Glu Glu
                85                  90                  95

Ala Lys Gly Leu Asp Arg Ile Asn Glu Arg Met Pro Pro Arg Arg Asp
            100                 105                 110

Ala Met Pro Ser Asp Ala Asn Leu Asn Ser Ile Asn Lys Ala Leu Thr
        115                 120                 125

Ser Glu Thr Asn Gly Thr Asp Ser Asn Gly Ser Asn Ser Ser Asn Ile
    130                 135                 140

Gln
145

<210>  13

<211>  23

<212>  DNA

<213>  Artificial

<220>

<221>  misc_feature

<223>  Primer for Calcineurin CNA-s1

<400>  13
atctgctcag acgatgaact rgg                                              23

<210>  14

<211>  21

<212>  DNA

<213>  Artificial

<220>

<221>  misc_feature

<223>  Primer for Calcineurin CNA-as1

<400>  14
ggcatcctct cgttaattcg g                                                21

<210>  15

<211>  31

<212>  DNA

<213>  Artificial

<220>

<221>  misc_feature

<223>  Primer for Calcineurin CNA-s2

<400>  15
catgccatgg atccatgtcc gagcccaagg c                                     31

<210>  16

<211>  46

<212>  DNA

<213>  Artificial

<220>

<221>  misc_feature

<223>  Primer for Calcineurin CNA-as4

<400>  16
tccccccggg gtaccctagt taatcactga atattgctgc tattac                     46

## Description

The present invention relates to a regulator of Calcineurin and uses of this regulator.

Calcineurin is a serine/threonine protein phosphatase, which is a heterodimer composed of a catalytic subunit (Calcineurin A) and a regulatory subunit (Calcineurin B). The catalytic subunit is about 60 and the regulatory subunit is about 18 kDa. This protein phosphatase is under the control of calcium and calmodulin, wherein binding of calcium and calmodulin is necessary for enzymatic activity.

Both subunits, the catalytic subunit and the regulatory subunit, occur in different isoforms which are encoded by different genes. In mammals, three distinct genes (A-α, A-β, A-γ) for the catalytic subunit have been characterized. In general, this subunit has five distinct domains: the N-terminal domain of sofar unknown function, the catalytic domain, the B-subunit binding domain, the calmodulin-binding domain and the autoinhibitory domain.

Calcineurin plays an important role in the coupling of calcium signals to cellular responses. Although it is ubiquitously expressed, distinct tissue expression patterns have been described for various isoforms of each subunit. Now Calcineurin is discussed in various contextes. For example, Calcineurin has been implicated in neuronal signalling pathways but the neuronal function is only poorly understood. Furthermore, Calcineurin is discussed in the context of immunosuppression, wherein Calcineurin acts via the transcription factor NFAT (Nuclear Factor Of Activated T-Cells) on the T-cell response.

Regarding the regulation of Calcineurin activity it is known that this enzyme is regulated by calcium and calmodulin (Rusnak, F. and Mertz, P. (2000), Physiol. Rev. 80, 1483-1521). It becomes activated by binding calcium and calmodulin when cytosolic calcium rises, and is inhibited on binding the immunosuppressants Cyclosporin A and FK 506 in complexes with their respective cellular receptors. Furthermore, Calcineurin regulators of the so-called calcipressin family have been described (Kingsbury, T.J. and Cunningham, K.W. (2000), Genes and Dev. 14, 1595-1604). Additionally, the cellular redox state has been implicated in the regulation of Calcineurin (Wang et al. (1996) Nature 383, 434-437).

Changes in Calcineurin expression, activity and tissue distribution have been described in a variety of disorders ranging from ischemia/reperfusion injury to cardiac hypertrophy, heart failure, stroke, amyotrophic lateral sclerosis, a degenerative motor neuron disease, skeletal muscle diseases and epilepsy.

Because Calcineurin plays a crucial role in widespread diseases like heart failure and stroke, there is a special interest in the regulatory mechanisms concerning the activity of Calcineurin. The detailed knowledge of the regulation mechanisms would provide a possibility to influence the activity of Calcineurin, especially in pathological situations. Thus, the invention has the object to provide suited targets for influencing the activity of this crucial enzyme and to provide active agents for the treatment of diseases, which are connected with a disturbance of Calcineurin activity.

This object is solved by a regulator of Calcineurin as described in claim 1. Preferred embodiments of this regulator are described in the dependent claims 2 - 4. The corresponding nucleotide sequences and amino acid sequences are claimed in the claims 5 - 8. In claims 9 - 13 an active agent for the treatment of diseases is described. Claims 14 - 16 relate to a use of said active agent a method for the treatment of diseases and a pharmaceutical composition. Claims 17 - 22 concern the use of the regulator for diagnosis of diseases and a corresponding kit. Claims 23 - 25 describe a method for searching substances, and the resulting active agent. The wording of all claims is hereby made to the content of the specification by reference.

The invention comprises a regulator of Calcineurin, which is based on a splice variant of the catalytic subunit of Calcineurin. In comparison with the complete subunit this regulator lacks the complete subunit, the catalytic and the Calcineurin B binding domain, therefore it possesses no phosphatase activity on its own. It retains part of the N-terminal domain, and the complete calmodulin-binding domain as well as the autoinhibitory domain. This inventive regulator is able to stimulate the phosphatase activity of Calcineurin, especially at low calcium concentrations. The splice variant itself binds calcium. Therefore the stimulating activity of the splice variant is especially surprising, because it should be assumed, that the splice variant and Calcineurin compete for calcium.

In a preferred embodiment of the invention the splice variant forms a protein complex with Calcineurin, especially with the catalytic Calcineurin holoenzyme. Especially preferred is a protein complex with Calcineurin and superoxide dismutase.

In general, the stimulating effect of the splice variant is dependent on low calcium concentrations. Preferably, these calcium concentrations are below 1 mM. A putative function of the splice variant under natural conditions is to render Calcineurin into a more calcium sensitive enzyme thus triggering Calcineurin linked signalling pathways at even lower calcium concentrations. The inventive splice variant provides an additional regulatory mechanism that ensures Calcineurin activity even at low calcium concentrations.

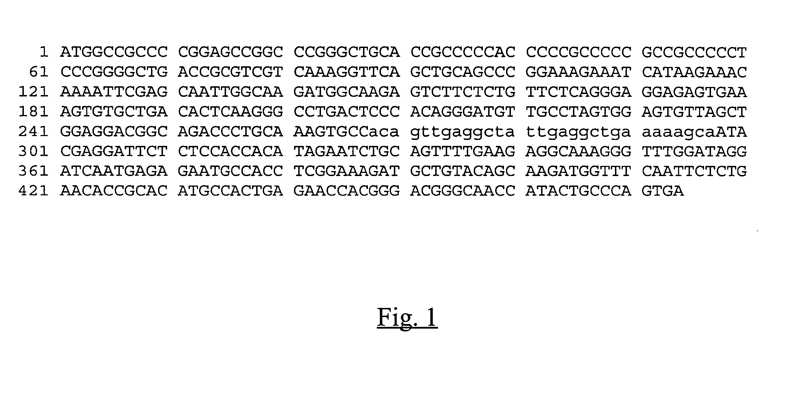

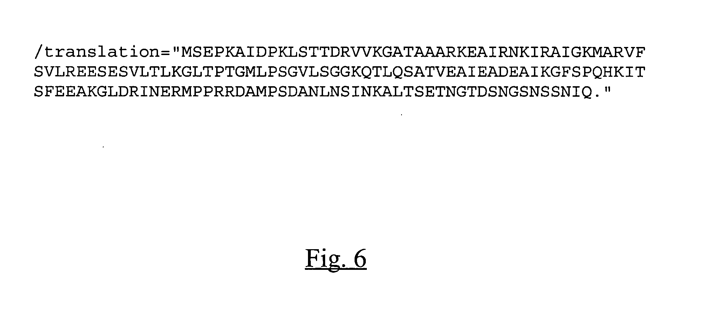

As outlined above, there are three isoforms of the catalytic subunit of Calcineurin. In a preferred embodiment of the invention the regulator of Calcineurin is based on a splice variant of the β-form, γ-form and/or α-form of the catalytic subunit. The splice variants of the different isoforms occur each in two forms (a) and (b), wherein one form (b) lacks 30 nucleotides respectively 10 amino acids as depicted in figures 1 to 6. In an especially preferred embodiment the regulator is characterized by an amino acid sequence at least 70 % and preferably at least 80 %, especially at least 90 % identical to the amino acid sequence of the splice variant of the β-form (SEQ ID NO 7, 8), the γ-form (SEQ ID NO 9, 10) and/or the α-form (SEQ ID NO 11, 12). Preferably, the inventive regulator is coded by a nucleotide sequence at least 70 % and preferably at least 80 %, especially at least 90 % identical to the nucleotide sequence coding for the splice variant of the β-form (SEQ ID NO 1, 2), the γ-form (SEQ ID NO 3, 4) and/or the α-form (SEQ ID NO 5, 6).

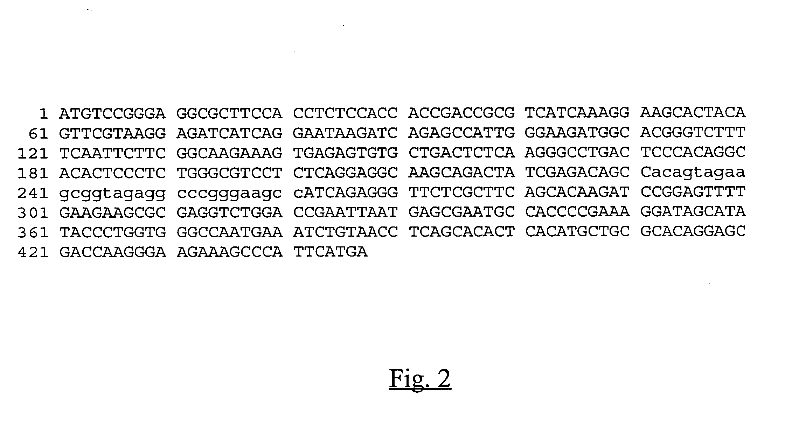

Furthermore, the invention comprises a nucleotide sequence, which is at least 70 % and preferably at least 80 %, especially at least 90 % identical to a nucleotide sequence according to figure 1 (SEQ ID NO 1, 2), which represents the sequence of the splice variant of the β-form, and/or which is at least 70 % and preferably at least 80 %, especially at least 90 % identical to a nucleotide sequence according to figure 2 (SEQ ID NO 3, 4), which represents the sequence of the γ-form of the splice variant. Preferably, these nucleotide sequences code for a regulator of Calcineurin.

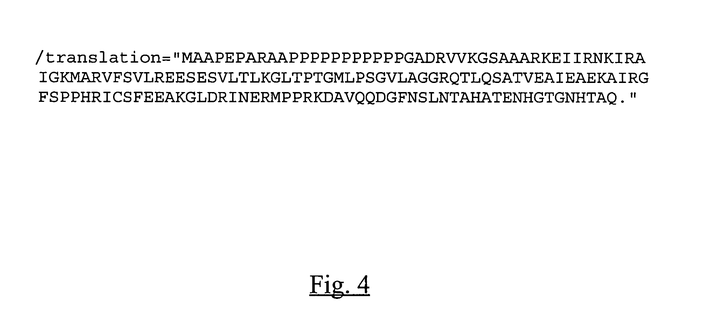

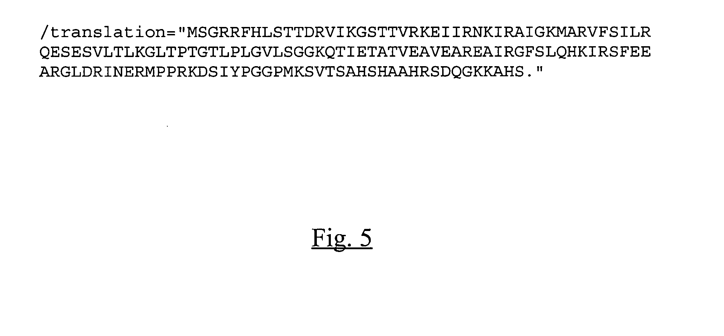

Additionally, the invention comprises an amino acid sequence, which is at least 70 % and preferably at least 80 %, especially at least 90 % identical to an amino acid sequence according to figure 4 (SEQ ID NO 7, 8), which represents the amino acid sequence of the β-form of the splice variant, and/or according to figure 5 (SEQ ID NO 9, 10), which represents the amino acid sequence of the γ-form of the splice variant. Preferably these amino acid sequences form a regulator for Calcineurin as described above.

Furthermore, the invention comprises an active agent especially for the treatment of diseases, wherein the active agent is the regulator of Calcineurin as described above. Since the inventive regulator is able to stimulate the phosphatase activity of Calcineurin, the active agent is especially useful for the treatment of diseases, which are accompanied by reduced Calcineurin activity, e.g. Alzheimers disease or amyotrophic lateral sclerosis.

In another embodiment of the invention an active agent for the treatment of diseases is provided, which influences the regulator of Calcineurin as it is described above. In one embodiment said active agent increases the activity of the regulator of Calcineurin. Such an active agent is especially useful for the treatment of diseases, which are accompanied by low Calcineurin activities. In another embodiment the active agent reduces the activity of the regulator of Calcineurin. Such an active agent is especially useful for the treatment of diseases which are accompanied by an increased Calcineurin activity.

Preferably the active agent is a substance, which interacts with the regulator thereby blocking its stimulating activity. In another embodiment the active agent is an inactive variant of the regulator, which competes for substrates with the functional regulator, and/or influences the forming of functional protein complexes. Such functional protein complexes may be built of Calcineurin and the regulator and/or superoxide dismutase, and/or other components.

One example of diseases, which could be effectively treated with an active agent, which reduces the activity of the regulator of Calcineurin, are cardiovascular diseases. For example in tissue of heart failure organs it is described, that Calcineurin is about four times higher than in normal tissue. By administering an active agent, which reduces the activity of a stimulator, i.e. the regulator of Calcineurin as described above, the pathological condition is counteracted.

In another embodiment of the invention the part of the regulator of Calcineurin is stimulation of Calcineurin, which results in the closing of calcium channels in a feedback mechanism, which terminates the activity of Calcineurin, because the necessary calcium influx is stopped. By adding an inventive agent, which reduces the activity of the regulator, i.e. the stimulator of Calcineurin, the feedback mechanism is stopped. Consequently, the calcium channels stay open and the calcium influx continues, resulting in a maintenance of Calcineurin activity. Consequently it depends on the certain circumstances of the disease to be treated, if an active agent is chosen, which increases the activity of the regulator of Calcineurin or an active agent is chosen, which reduces said activity. In general, cardiovascular diseases and/or neurodegenerative diseases like epilepsy or amyotrophic lateral sclerosis and other diseases like skeletal muscle diseases, for example, are preferred candidates for the treatment with an active agent according to the invention.

Furthermore, the invention comprises the use of an active agent as described above for the treatment of diseases, and a method for the treatment of diseases, wherein at least one inventive active agent as described above is administered. Regarding further details of said use and said method reference is made to the description above.

Additionally, the invention comprises a pharmaceutical composition, which comprises at least one active agent as described above in an effective amount and at least one pharmaceutical carrier. Depending from the circumstances of the disease to be treated the pharmaceutical composition is intended for topical or systemical administration, for example.

Furthermore, the invention comprises the use of a regulator of Calcineurin for the diagnosis of diseases, wherein the frequency of said regulator in the organism is analysed. As outlined above, several disorders are accompanied by an increased or decreased activity of Calcineurin in comparison with non-pathological conditions. In many cases this altered Calcineurin activity is caused by a changed activity and/or frequency of the regulator of Calcineurin, which is based on a splice variant as described above. For example in an animal model for amyotrophic lateral sclerosis, the inventive splice variant of the β-form of Calcineurin A is considerably stronger expressed than in normal animals, as described in further detail in the examples below. Therefore the invention comprises the use of the regulator for diagnosis of the respective diseases, especially in the early diagnosis of the diseases. In a preferred embodiment of the inventive use at least one substance is used, which interacts with the regulator of Calcineurin. The regulator of Calcineurin is preferably a regulator as described above.

In a preferred embodiment of the inventive use said interacting substance is an antibody, which interacts with the regulator, preferably on the level of protein-protein or protein-peptide interaction. In general, any antibody specific for the regulator is suitable in the inventive manner, for example polyclonal antibodies and/or monoclonal antibodies, as will be obvious for an expert in the art. In a preferred embodiment of this inventive use the specific antibody is placed on an array, which permits a fast and easy performance of the diagnostic test, for example the antibody is placed on a dip stick as it is known in the field of immunodiagnosis.

In another embodiment of the inventive use the interacting substance is a nucleic acid molecule, which hybridizes with at least a portion of a nucleic acid sequence coding for the regulator. This embodiment is especially preferred, because such hybridisation probes are cheap and easy to obtain and the diagnostic test can be performed by common methods. For example the hybridisation probe, i.e. the nucleic acid molecule, which hybridizes with at least a portion of the nucleic acid sequence coding for the regulator, is placed on a DNA chip, and the complete diagnostic reaction is preferably performed in an automated manner. In this embodiment the inventive use is especially useful for fast diagnosis of different diseases.

Preferred examples of said diseases are connected with a disturbance of Calcineurin activity, for example said diseases are neurodegenerative diseases like amyotrophic lateral sclerosis and/or said diseases are cardiovascular diseases like heart failure.

Furthermore, the invention comprises a kit, which comprises at least one substance, which is able to interact with the regulator of Calcineurin. This kit is especially useful for diagnosis as described above. In a preferred embodiment of said kit the kit comprises at least one specific antibody for the regulator. In another embodiment of the kit the kit comprises at least one nucleic acid molecule, which is able to hybridize with at least a portion of a nucleic acid sequence coding for the regulator.

Furthermore, the invention comprises a method for searching substances, which influence the activity of Calcineurin. This method is characterized in that a splice variant of the catalytic subunit of Calcineurin is used to identify and/or isolate substances, which are able to interact with the splice variant itself and/or with Calcineurin. In a preferred embodiment of this method the activity of Calcineurin is measured in vitro, whereby the influence of the regulator is analysed with or without (as control) additional substances, which possibly influence the activity of Calcineurin via the regulator. In another preferred embodiment of this method the complex formation between at least Calcineurin, the regulator and the substance possibly influencing the activity of Calcineurin is used as read-out system. Regarding further details of the regulator used in this method reference is made to the description above.

Finally, the invention comprises an active agent for the treatment of diseases, which is identified and/or isolated by a method as described above.

The described features and further features of the invention read in greater detail from the following description of the figures in conjunction with the examples and the subclaims, whereby each of the individual features could be realized alone or in combination with each other.

In the figures it is shown:
Figure 1: Nucleotide sequence of the inventive splice variant of the β-form of Calcineurin A, a further variant of this splice variant lacks nucleotides 268 to 297,
Figure 2: Nucleotide sequence of the inventive splice variant of the γ-form of Calcineurin A, a further variant of this splice variant lacks nucleotides 232 to 261,
Figure 3: Nucleotide sequence of the inventive splice variant of the α-form of Calcineurin A, a further variant of this splice variant lacks nucleotides 244 to 273,
Figure 4: Amino acid sequence of the inventive splice variant of the β-form of Calcineurin A, a further variant of this splice variant lacks amino acid residues 90 to 99,
Figure 5: Amino acid sequence of the inventive splice variant of the γ-form of Calcineurin A, a further variant of this splice variant lacks amino acid residues 78 to 87,
Figure 6: Amino acid sequence of the inventive splice variant of the α-form of Calcineurin A, a further variant of this splice variant lacks amino acid residues 82 to 91,
Figure 7: PCR amplification of Calcineurin A-α. Calcineurin cDNAs were amplified by nested PCR and the amplicons were separated on a 0,8 % agarose gel. In addition to the known transcripts at 1.6 kb, an additional splice variant of about 450 bp was detected,
Figure 8: Domain and exon structure of Calcineurin (CN) Aα16K. The sequence consists of exons 1, 11, 12, 13 and 14 and contains part of the N-terminal domain, the calmodulin-binding domain and the autoinhibitory domain. Catalytic domain (exon 2 - 8) and Calcineurin B-binding domain (exon 9 + 10) are missing. CN B: Calcineurin B-binding domain; CaM: calmodulin-binding domain; Al: autoinhibitory domain,

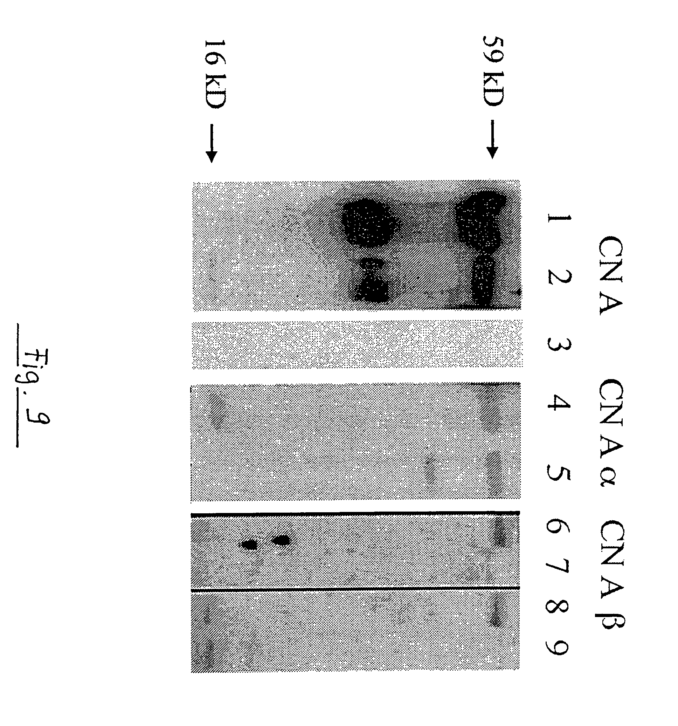

Figure 9: Immunoblot of Calcineurin. 1: bovine brain Calcineurin (Sigma), 2: bovine brain Calcineurin pulled down with Cu/Zn superoxide dismutase (Cu/Zn SOD was bound to NiNTA-magnetic beads prior to incubation with bovine brain CNA, Sigma). 3: human recombinant SOD. 4-9: murine spinal cord protein extracts, 4^{th}, 6^{th} and 8^{th} lane: cytosolic fraction, 5^{th}, 7^{th} and 9^{th} lane: pellet fraction, 4^{th} - 7^{th} lane: wild-type. 8^{th} and 9^{th} lane: mice transgenic for human mutated Cu/ZnSOD. 1 to 3 were visualized with a monoclonal mouse anti-Calcineurin A antibody, 4 and 5 were visualized with a rabbit anti-Calcineurin Aα, 6-9 with a rabbit anti-Calcineurin Aβ antibody (Chemicon, Temecula, USA).
Figure 10: The activation of phosphatase activity by CNAα16K is calcium (Ca)-dependent. Calcineurin assays were performed as described below. Different concentrations of free Ca were reached by addition of EDTA. Values are given as mean +/- standard deviation. N=7. **p<0.01, significantly different from control CN activity by Student's t test,
Figure 11: Preincubation increases the efficiency of CNAα16K. Calcineurin activity assays were performed as described below. Preincubation indicates that standard CN and CNAα16K were added and incubated for 30 minutes before addition of substrate, whereas without preincubation both proteins were added immediately before adding substrate. Values are given as mean +/- standard deviation. N=7. *p<0.02, significantly different from control CN activity by Student's t test; **p<0.01, significantly different from control CN activity by Student's t test,

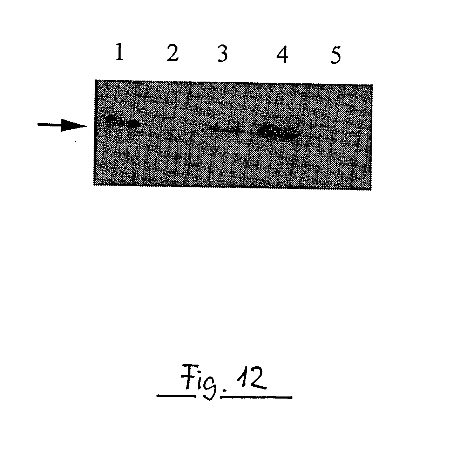

Figure 12: Direct interaction of Calcineurin Aα16K and standard Calcineurin. 1: Calcineurin positive control. 2: bacterial control eluate was bound to Ni-NTA magnetic beads and used to pull down bovine brain CN (Sigma). 3: pull-down of Calcineurin with CNAα16K (CNAα16K was bound to Ni-NTA-magnetic beads prior to incubation with bovine brain CN, Sigma). 4: pull-down of spinal cord homogenate with CNAα16K (CNAα16K was bound to NiNTA-magnetic beads prior to incubation with murine brain homogenate). 5: bacterial control eluate was bound to Ni-NTA-magnetic beads and used to pull down spinal cord homogenate.

### Examples:

In the following description of examples it is shown the cloning and biochemical characterization of the inventive splice variant of the α-form of Calcineurin A. This novel short splice variant lacks the catalytic and the Calcineurin B binding domain. The translation product of this short cDNA, which comprises of exons 1 and 11 - 14, was detected in the aspected size range by Calcineurin A-positive immunoreactivity in western blots of Calcineurin pulled down by superoxide dismutase and in murine spinal cord extracts. Using different antibodies which recognize epitopes in the N-terminal region of the other isoforms of Calcineurin A, corresponding short Calcineurin variants were detected in spinal cord of wild-type mice. The inventive splice variants were especially detected in membrane fractions, and in particular in membrane fractions of the nucleus.

However, although lacking any phosphatase activity on its own, addition of these splice variants to standard Calcineurin increases the phosphatase activity of Calcineurin. This stimulating effect is Ca-dependent, for it is seen only in low calcium concentrations. A possible function of the splice variants therefore is to render Calcineurin into a more calcium sensitive enzyme thus triggering Calcineurin linked signalling pathways, for instance transcription activation through NF-AT, at even lower calcium concentrations. As a result, Calcineurin regulated feedback mechanisms like dephosphorylation and thereby closing of glutamate receptors are likely to reduce toxic calcium influxes. For example with respect to amyotrophic lateral sclerosis, the inventive splice variants are able to enhance the susceptibility of cells to calcium-influx and therefore are at least partially responsible for influences on calcium-linked signalling in amyotrophic lateral sclerosis as well as in apoptosis of neurons, stroke and heart failure, for example.

As shown later on, the stimulating effect of the inventive splice variants increases when a splice variant is preincubated with Calcineurin. This shows a time-dependent mechanism, which is also strenghtened by the detection of a direct physical interaction between the splice variant and standard Calcineurin. Possibly the effect of the inventive splice variants works via calmodulin, e.g. increasing the activating effect of calmodulin by stabilizing the Calcineurin-calmodulin complex.

### 1. Materials and methods

### 1.1 Calcineurin Aα cloning

Calcineurin Aα was amplified by a nested PCR from human spinal cord poly-A⁺-RNA from Clontech, Palo Alto, California. PCRs were conducted in a 25 µl volume. They contained 2.5 µl 10x PCR-buffer (Stratagene), 0.25 µl 25mM dNTP-mix, 0.25 µl CNA-s1 primer (10 µM), 0.25 µl CNA-as1 primer (10 µM), 2.5 µl cDNA, 0.25 µl Taq Polymerase (Promega), 0.25 µl Pfu polymerase and distilled water ad 25 µl. PCR was started by a first heating step at 95°C for 3 min. The amplification conditions were 35 cycles of 40 sec at 95 °C for denaturing, 40 sec at 55 °C for annealing and 3 min at 72 °C for elongation. PCR was terminated with a 10 min extension step at 72 °C.

The nested PCR was performed with 2.5 µl of the first amplification reaction with the primers CNA-s2 and CNA-as4 as above. The amplification conditions were 25 cycles of 40 sec at 95 °C for denaturing, 40 sec at 55 °C for annealing and 3 min at 72 °C for elongation. PCR was terminated with a 10 min extension step at 72 °C.

Primer sequences were:

CNA-s1: ATC TGC TCA GAC GAT GAA CTR GG (SEQ ID NO 13)

CNA-as1:   GGC ATC CTC TCG TTA ATT CGG   (SEQ ID NO 14)

CNA-s2: CAT GCC ATG GAT CCA TGT CCG AGC CCA AGG C
(SEQ ID NO 15)

CNA-as4: TCC CCC CGG GGT ACC CTA GTT AAT CAC TGA ATA
TTG CTG CTA TTA C (SEQ ID NO 16)

The resulting amplicons were digested with Bam HI and Xma I, electrophoresed and purified after gel electrophoresis with the Qiaquick gel extraction kit (Qiagen, Hilden, Germany) according to the manufacturers recommendations. The digested amplicons were ligated into pQE30 vector (Qiagen, Hilden, Germany) digested with Bam HI and Xma I. Resulting clones were sequenced completely.

### 1.2 Expression and purification of CNAα16K clones

The resulting plasmid pQE30K16 was transformed into M15 bacteria, positive clones were inoculated into LB medium containing 50µg/ml Ampicillin at 37°C overnight with vigorous shaking. Next morning 0.5 ml bacteria were inoculated into 1 liter LB medium containing 100µg/ml ampicillin, after the bacteria reached a OD₆₀₀ Of 0.5 to 0.7, IPTG was added to the culture to a final concentration of 1 µmol/l. After 4 hours the bacteria were pelleted by centrifugation at 5,000g for 20 min and the pellet was frozen at -80 °C over night.

The frozen pellet was thawed in ice for 15-30 min and the cells were resuspended in lysis buffer (50mM NaH₂PO₄, 300mM NaCI (pH8.0)) at 2.5 ml per gram wet weight. Lysozyme was added for a final concentration of 1 mg/ml and the cells were incubated on ice for 30 min, then the cells were sonicated on ice 6 times for 10 seconds. Cellular debris was pelleted by a centrifugation of 30 min at 10,000g and 4 °C and the supernatant was mixed with 1 ml of a 50 % slurry of Ni-NTA.

The mixture was incubated at 4 °C with gentle shaking for 60 min and loaded into a column.

After washing the column 3 times with 4 ml of wash buffer (50mM NaH₂PO_{4,} 300mM NaCI, 50mM imidazole (pH8.0)) the protein was eluted with 1.6 ml elution buffer (50mM NaH₂PO₄, 300mM NaCl, 500mM imidazole (pH8.0)). After elution, a buffer exchange was performed by ultrafiltration (centrex UF-2, Schleicher & Schuell, Dassel).

### 1.3 Western blotting

After SDS-PAGE on 12% polyacrylamide gels, protein was transferred to nitrocellulose membranes according to standard protocols. Immunodetections were performed with a monoclonal mouse anti-Calcineurin A antibody from Sigma (dilution 1:4000), polyclonal rabbit anti-Calcineurin A beta and alpha antibodies from Chemicon (dilution 1:1000) and secondary HRP-conjugated sheep anti-mouse and donkey anti-rabbit antibodies from Amersham Pharmacia (dilution 1:1000) according to the manufacturers recommendations. Detection was performed with the ECL plus kit from Amersham Pharmacia.

### 1.4 Calcineurin activity assay

Calcineurin activity was assayed with the Calcineurin Activity Assay kit (Calbiochem, Palo Alto, USA). In short, after desalting the Calcineurin with a desalting column, 5µl purified Calcineurin (Sigma Aldrich, Deisenhofen, Germany) were added to 25 µl 2x assay buffer (100mM NaCI, 50mM Tris, 6mM MgCl₂, 0.5mM CaCl₂, 0.5mM DTT, 0.25% NP-40, 0.25µM Calmodulin), 10 µl substrate solution (150µM RII Phosphopeptide) with or without 5 µl CNAα16K solution (1.5 µg/µl). After 30 minutes the reaction was stopped by addition of 100 µl of Green reagent and after additional 15 minutes developing time the absorption at 260 nm was measured. Released phosphate was calculated using a phosphate standard curve. For the detection of calcium sensibility of the activity, EDTA was added to the 2x assay buffer to final concentrations of 5 mM (free calcium: 5 µM) and 1.5 mM (free calcium: 65 µM). Calculation of free calcium was performed by WEBMAXC v. 2.1 (Stanford, USA).

### 1.5 Pull-down assay

After purification of CNAα16K, a buffer exchange by ultrafiltration was performed to get rid of imidazole. For the assay, 50 µg of CNAα16K in buffer A (50mM NaH₂PO₄, 300mM NaCI, pH8.0) were incubated with 50 µl of Ni-NTA magnetic beads (Qiagen, Hilden, Germany) and 300 µl of buffer A with or without the addition of 10 µg of purified bovine brain Calcineurin (Sigma Aldrich, Deisenhofen, Germany) or 100 µl murine brain homogenate for 16 h at 4 °C. Corresponding eluate fractions from bacteria were transformed with pQE30 alone and treated exactly the same as the CNAα16K served as control for the CNAα16K.

After binding, the beads were washed 2 times for 30 minutes with 500 µl of buffer A at constant shaking (1000 rpm, **4** °C) and elution was performed with 100 µl of buffer C buffer (50mM NaH₂PO₄, 300mM NaCI, 500mM imidazole, pH8.0). The calcineurin was detected by western blotting.

### 1.6 Protein extracts

Frozen spinal cord tissues were transferred into 2x homogenization buffer (500 mM Sucrose, 4mM EDTA, 0.02 % β-mercaptoethanol; 500 µl/100 mg tissue). For protection against proteases 1/10 vol PMSF (10 mg/ml) and Leupeptin (1 mg/ml) were added respectively. Finally the volume was equilibrated to 1000 µl/100 mg tissue with distilled water. The tissues were homogenized with a glass homogenizer in this solution and then transferred into cups. These cups were centrifuged for 3 hours at 4 °C and 14,000 x g to separate soluble from particle (non-soluble) containing fractions. The soluble fraction was filled in a new cup after centrifugation. The particle containing cup was filled up with an adequate volume of 2x homogenization buffer. Protein content of both fractions was determined by the BioRad protein assay reagent, with bovine serum albumin as the standard.

### 2. Results

### 2.1 Characterization and structure of a cDNA for a novel Calcineurin Aα splice variant

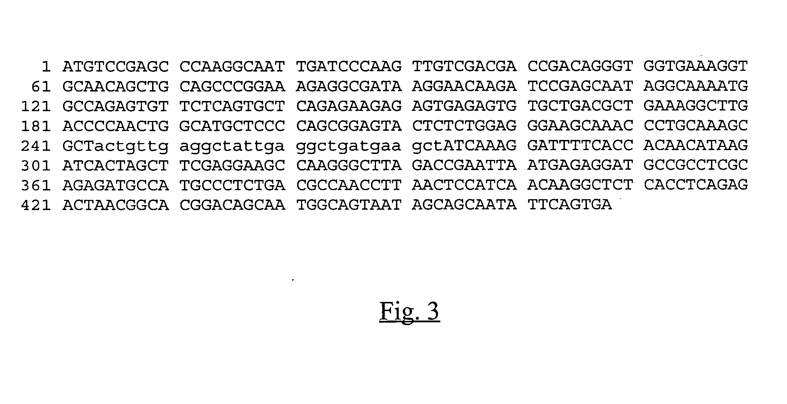

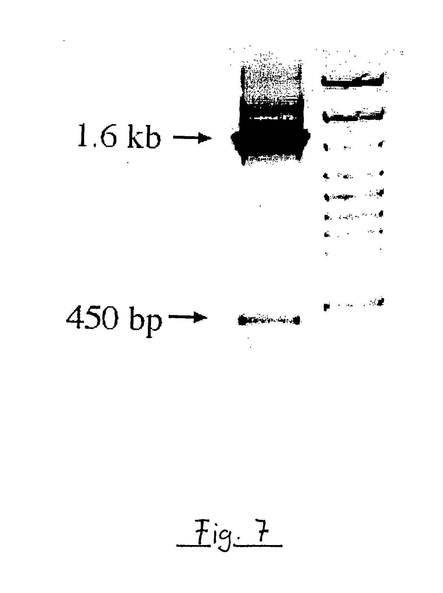

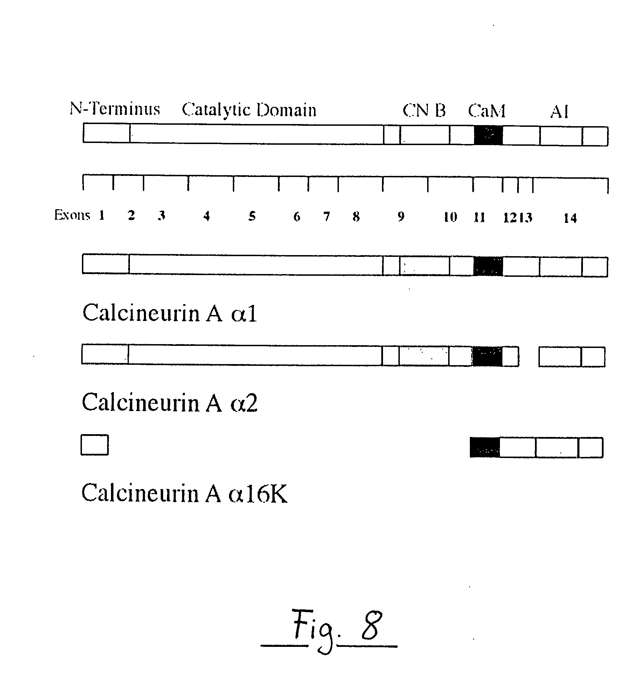

Calcineurin Aα splice variants were amplified by a nested PCR from human spinal cord poly-A⁺-RNA (Clontech, Palo Alto, USA). A strong band at 1.6 kb indicated the transcripts for the Calcineurin Aα splice variants. Cloning and sequencing indeed revealed that the 1.6 kb band consisted of two splice variants, differing by 30 base pairs by alternative splicing of exon 13. In addition, a smaller band was obtained at about 450 bp that was subsequently isolated and cloned (fig. 7). Sequencing revealed that this amplicon encompassed an open reading frame of 438 base pairs which showed 100% identity to the coding base pairs 1-57 and 1158-1533 of Calcineurin Aα, thereby representing the exons 1, 11, 12, 13 and 14 of the Calcineurin Aα gene (fig. 3). The cDNA, which was named CNAα16K, codes for a protein of 145 amino acids with a calculated molecular weight of 15,522 dalton, that contains part of the N-terminal domain and the calmodulin-binding domain as well as the autoinhibitory domain. Catalytic and Calcineurin B-binding domain from standard Calcineurin Aα missed completely (fig.8). In addition bioinformatic analysis shows a weak similarity to cystein proteases.

To determine whether this cDNA represents a cloning artefact or whether it is a novel Calcineurin Aα splice variant that is transcribed into protein, western blot analysis on purified, commercially available bovine Calcineurin (Sigma Aldrich, Deisenhofen, Germany) were perfomed. Although a smaller immunoreactive band was visible in the range of 35 kD with an anti-Calcineurin A antibody, immunoreactivity in the range of 16 kD was not detectable. In view of the fact that Calcineurin activity is protected by superoxide dismutase and also copurifies with this enzyme, a pull-down assay with purified Calcineurin and human recombinant superoxide dismutase (SOD) as binding partner was performed and the interacting proteins were analysed by western blotting. As can be seen in fig. 9, without SOD as binding partner only the standard Calcineurin and the already seen 35 kD band was detectable (lane 1), whereas in the assay with SOD as binding partner, an additional band at about 16 kD was detectable (fig. 9, lane 2). No Calcineurin immunoreactivity could be seen in the human recombinant SOD alone (lane 3). Obviously, CNAα16K was enriched through an affinity to human recombinant SOD.

However, when using an antibody directed against the N-terminal part of Calcineurin Aα, a smaller band at about 16 kD could be detected directly in the cytosolic fraction of spinal cord extracts of mice in addition to the 59 kD standard variant (fig. 9, lane 4), wehreas no 16 kD band was observable in the pellet fraction (fig. 9, lane 5).

In addition, when using an antibody directed against the N-terminal part of Calcineurin Aβ, immunoreactivity in the range of 16 kD was easily detectable in both cytosolic and pellet fractions of protein extracts from spinal cord of mice (fig. 9, lanes 6-9). Interestingly, expression of this short splice variant was considerably stronger in mice transgenic for mutated human superoxide dismutase developing a phenotype closely resembling human amyotrophic lateral sclerosis (fig. 9, lane 8 and 9), suggesting a role of the splice variant in motor neuron diseases.

### 2.2 Recombinant expression, purification and biochemical analysis of Calcineurin Aα16K

To analyse the new Calcineurin splice variant biochemically, the cDNA was cloned into the pQE30 expression vector, resulting in a hypothetical protein of 158 amino acids and a calculated molecular weight of 16,921.13 Da, with an N-terminal HIS-tag for quick purification from bacterial lysates. Induction with IPTG and purification with Ni-NTA agarose indeed yielded a protein of 17 kDa. Expression efficiency was low, from a 500 ml culture about 600 µg of Calcineurin Aα16K protein was yielded. Western-blotting of affinity column eluates revealed positive immunoreactivity with the anti-Calcineurin A antibody.

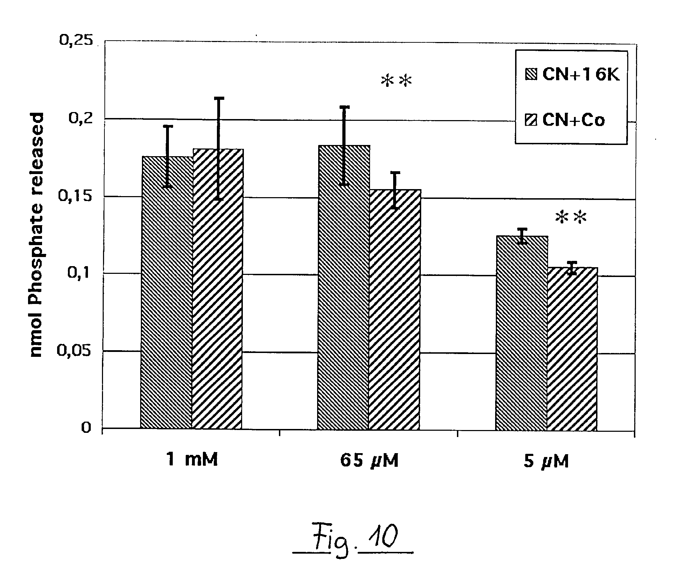

As expected by the lack of the catalytic domain, CNAα16K exhibited no phosphatase activity in a Calcineurin activity assay using phospho-RII peptide as a substrate. Secondly, the influence of CNAα16K on CN A activity was investigated by incubating purified bovine Calcineurin (Sigma, Deisenhofen) with and without CNAα16K. As a control, bacteria were transformed with pQE30, induced, harvested and the purification process was repeated exactly as in the case of the Calcineurin Aα16K. Comparable elution fractions served as controls for the CNAα16K. As seen in fig. 10, addition of purified CNAα16K to purified Calcineurin holoenzyme stimulated the dephosphorylation of RII peptide when compared to addition of control eluate. In comparison to addition of control eluate (100%), the phosphatase activity of Calcineurin increased to 118.5% (65 µM free Ca) and 119.6% (5 µM free Ca). Interestingly, this effect could only be seen in low calcium concentrations (fig. 10). In calcium concentrations under standard assay conditions, usually in the range of 1 mM, no significant effect could be detected, indicating that activation of phosphatase activity by CNAα16K is essentially restricted to low but physiological relevant calcium concentrations.

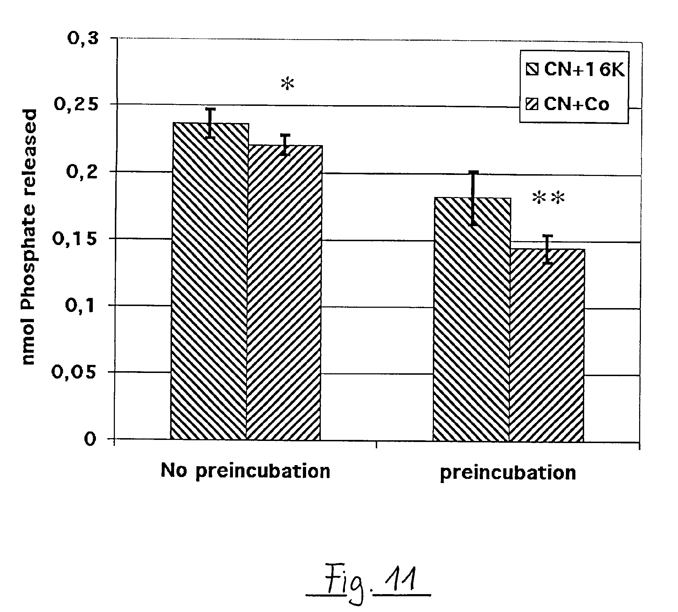

A preincubation of Calcineurin with CNAα16K for 30 minutes before adding the substrate increased the activating effect of CNAα16K (126 % of control activity) compared with direct addition of CNAα16K to Calcineurin immediately before measurement (106 % compared to control activity, fig. 11).

A pull down assay with CNAα16K as binding protein was performed, to verify if there is a direct interaction in vitro between CNAα16K and purified Calcineurin. In this assay, CNAα16K was bound to magnetic Ni-NTA beads and incubated with purified bovine Calcineurin as well as murine spinal cord eluates. Again, the corresponding eluates from pQE30-transformed and Ni-NTA agarose purified bacteria served as controls. As seen in fig. 12, strong binding of Calcineurin to immobilized histidine-tagged CNAα16K could be seen either using purified bovine brain Calcineurin or murine spinal cord homogenate (fig. 12, lanes 3 and 4). In contrast, no detectable binding was seen where the control eluate was used (fig. 12, lanes 2 and 5).

## Claims

1. Regulator of Calcineurin, **characterized in that** it is based on a splice variant of the catalytic subunit of Calcineurin.

2. Regulator of Calcineurin according to claim 1, **characterized in that** the catalytic subunit is the β-form, γ-form and/or α-form of said subunit.

3. Regulator of Calcineurin according to claim 1 or claim 2, **characterized by** an amino acid sequence at least 70 % identical to an amino acid sequence according to figure 4 (SEQ ID NO 7, 8), figure 5 (SEQ ID NO 9, 10) and/or figure 6 (SEQ ID NO 11, 12).

4. Regulator of Calcineurin according to one of the preceding claims, **characterized in that** it is coded by a nucleotide sequence at least 70 % identical to a nucleotide sequence according to figure 1 (SEQ ID NO 1, 2), figure 2 (SEQ ID NO 3, 4) and/or figure 3 (SEQ ID NO 5, 6).

5. Nucleotide sequence, **characterized in that** it is at least 70 % identical to a nucleotide sequence according to figure 1 (SEQ ID NO 1, 2) and/or figure 2 (SEQ ID NO 3, 4).

6. Nucleotide sequence according to claim 5, **characterized in that** it codes for a regulator of Calcineurin.

7. Amino acid sequence, **characterized in that** it is at least 70 % identical to an amino acid sequence according to figure 4 (SEQ ID NO 7, 8) and/or figure 5 (SEQ ID NO 9, 10).

8. Amino acid sequence according to claim 7, **characterized in that** it forms a regulator for Calcineurin.

9. Active agent especially for the treatment of diseases, **characterized in that** the active agent is a regulator according to one of claims 1 to 4.

10. Active agent especially for the treatment of diseases, **characterized in that** the active agent influences a regulator according to one of claims 1 to 4.

11. Active agent according to claim 10, **characterized in that** the active agent increases the activity of said regulator.

12. Active agent according to claim 10, **characterized in that** the active agent reduces the activity of said regulator.

13. Active agent according to one of claims 9 to 12, **characterized in that** said diseases are connected with a disturbance of Calcineurin activity, wherein preferably said diseases are neurodegenerative and/or cardiovascular diseases.

14. Use of an active agent according to one of claims 9 to 13 for the treatment of diseases.

15. Method for the treatment of diseases, **characterized in that** at least one active agent according to one of claims 9 to 13 is administered.

16. Pharmaceutical composition, **characterized in that** it comprises at least one active agent according to one of claims 9 to 13 in an effective amount and at least one pharmaceutical carrier.

17. Use of a regulator of Calcineurin for diagnosis of diseases, **characterized in that** the frequency of said regulator is analysed, wherein preferably at least one substance is used, which interacts with the regulator.

18. Use according to claim 17, **characterized in that** said regulator is a regulator according to one of claims 1 to 4.

19. Use according to claim 17 or claim 18, **characterized in that** said interacting substance is an antibody against the regulator.

20. Use according to one of claims 17 to 19, **characterized in that** said interacting substance is a nucleic acid molecule, which hybridizes with at least a portion of a nucleic acid sequence coding for the regulator.

21. Use according to one of claims 17 to 20, **characterized in that** said diseases are connected with a disturbance of Calcineurin activity, wherein preferably said diseases are neurodegenerative and/or cardiovascular diseases.

22. Kit for diagnosis of diseases, comprising at least one substance, which interacts with a regulator according to one of claims 1 to 4.

23. Method for searching substances influencing the activity of Calcineurin, **characterized in that** a splice variant of the catalytic subunit of Calcineurin is used to identify and/or isolate substances interacting with the splice variant and/or Calcineurin.

24. Method according to claim 23, **characterized in that** the splice variant is a regulator according to one of claims 1 to 4.

25. Active agent for the treatment of diseases, **characterized in that** the active agent is identified and/or isolated by a method according to claim 23 or claim 24.
